# EUROPEAN PATENT SPECIFICATION

(11) **EP 4 526 404 B1**
(45) Date of publication and mention of the grant of the patent: **24.12.2025**
(21) Application number: 23727366.9
(22) Date of filing: 17.05.2023
(51) Int. Cl.: C10G 2/00, C10G 3/00

(54) **METHOD FOR PRODUCING SUSTAINABLE FUEL VIA METHANOL**
VERFAHREN ZUR HERSTELLUNG VON NACHHALTIGEM KRAFTSTOFF MITTELS METHANOL
PROCÉDÉ DE PRODUCTION DE CARBURANT DURABLE PAR L'INTERMÉDIAIRE DE MÉTHANOL

(30) Priority: 19.05.2022 EP 22174409
(43) Date of publication of application: 26.03.2025
(73) Proprietor: TotalEnergies OneTech, 92400 Courbevoie (FR)
(72) Inventor: STEWART, Joseph, 7181 Seneffe (BE); VERMEIREN, Walter, 3530 Houthalen (BE)
(74) Representative: Synergy IP Group AG
(86) International application number: PCT/EP2023/063327
(87) International publication number: WO 2023/222799

(56) References cited:
- EP-A1- 3 670 443
- EP-A2- 1 390 443
- US-A- 5 344 849
- US-A1- 2006 211 777
- NEZAM I ET AL: "Direct aromatization of CO2via combined CO2hydrogenation and zeolite-based acid catalysis", JOURNAL OF CO2 UTILIZATION 20210301 ELSEVIER LTD GBR, vol. 45, 20 January 2021 (2021-01-20), pages 1 - 21, XP002807864
- RICHTER SANDRA ET AL: "Paths to alternative fuels for aviation", CEAS AERONAUTICAL JOURNAL, SPRINGER VIENNA, VIENNA, vol. 9, no. 3, 9 March 2018 (2018-03-09), pages 389 - 403, XP036573963, ISSN: 1869-5582, [retrieved on 20180309], DOI: 10.1007/S13272-018-0296-1

## Description

### Field of the invention

The invention relates to methods for producing sustainable fuel, and to respective sustainable fuels.

### Background of the invention

Climate change and the on-going energy transition makes it mandatory to replace fossil-based energy sources. In this context various aspects will be important for society to reach Net Zero by 2050 as currently desired. For example, the valorisation of alternative feedstocks is expected to contribute towards the circular economy and/or reduce the carbon dioxide (CO₂) footprint associated with the final product. However, while many alternative fuels and chemicals are sort after, drop in solutions from alternative feedstocks would allow for existing infrastructure to be maintained.

In this context even the valorisation of CO₂ as a feedstock is considered, sourced from flue gas, bio sources and even direct air capture. Upgrading of CO₂ to chemicals such as methanol has already received some attention to yield sustainable fuel for various applications. However, some applications regularly lead to enhanced requirements for the sustainable fuel. For example, aviation fuel, i.e., fuel to power aircraft, regularly requires to contain larger hydrocarbons and aromatics. This is because larger hydrocarbons and aromatics regularly improve the cold flow properties of the aviation fuel. Such improved cold flow properties prevent fuel freezing at low temperatures of for example -40°C, which are typical for cruising altitudes of the powered aircrafts. In attempts to upgrade CO₂, larger hydrocarbons and aromatics are however regularly more difficult to achieve.

In a different approach, bio conversion and syngas upgrading via Fischer-Tropsch (FT) are also considered as an alternative to a fossil fuel feedstock. Such routes may appear attractive for diesel and gasoline production. However, they are not suitable for 100% aviation fuel, as they crucially lack aromatic content and hence are applied as a blend.

Thus far, there are no established routes of producing a 100% sustainable aviation fuel by bio conversion or syngas upgrading via FT.

Further in the search for sustainable fuel and in particular in the search for aviation fuel, hydrodeoxygenation of fatty acids/triglycerides, CO₂- or bio-sourced syngas upgrading, or bio-olefin oligomerisation have been researched at various Technology Readiness Levels (TRLs). However, for each of these routes, the major product is regularly long hydrocarbon chains and is typically only suitable for up to 50% blend to meet requirements for aviation fuel. This is because these routes do regularly not yield aromatics which are however also required to improve the cold flow properties of aviation fuel. Nezam I: "Direct aromatization of CO2via combined CO2hydrogenation and zeolite-based acid catalysis", JOURNAL OF CO2 UTILIZATION 20210301 ELSEVIER LTD GBR, vol. 45, 20 January 2021 (2021-01-20), pages 1-21, discloses a method for producing aromatics from CO₂.

Overall, there remains a general desire for an improved method for producing sustainable fuel.

### Problem underlying the invention

It is an object of the present invention to provide a method for producing sustainable fuel which at least partially overcomes the drawbacks encountered in the art.

It is in particular an object of the present invention to provide a method for producing sustainable fuel which reduces the CO₂ footprint associated with the produced sustainable fuel.

It is furthermore an object of the present invention to provide a method for producing sustainable fuel which allows for existing infrastructure to be maintained.

It is moreover an object of the present invention to provide a method for producing sustainable fuel which has an improved energy efficiency and/or an improved cost efficiency.

It is also an object of the present invention to provide a method for producing sustainable fuel which has an improved carbon efficiency, i.e., a method which minimizes carbon dioxide equivalents emissions to its output.

It is additionally an object of the present invention to provide a method for producing sustainable fuel which has an increased aromatics content.

It is in particular an object of the present invention to provide a method for producing sustainable fuel which meets the requirements for aviation fuel.

### Disclosure of the invention

Surprisingly, it has been found that the problem underlying the invention is overcome by methods according to the claims. Further embodiments of the invention are outlined throughout the description.

Subject of the invention is a method for producing sustainable fuel, comprising the steps:
(i) converting a feed mixture comprising CO₂ into an intermediate mixture comprising CO and aromatics by
   (ia) converting the CO₂ at least partially into methanol and CO using a CO₂-to-methanol catalyst, and
   (ib) converting methanol from step (ia) at least partially into aromatics using a zeolite-based catalyst, and
(ii) converting CO from step (ia) at least partially into saturated hydrocarbons using a Fischer-Tropsch catalyst, wherein the saturated hydrocarbons comprise saturated C7+ hydrocarbons.

Logically, steps (i) and (ii) are carried out in the given order, i.e., first step (i) and thereafter step (ii). However, additional steps before or after each of steps (i) and (ii) may also be comprised by the method according to the present invention.

In step (i), CO₂ is comprised by a feed mixture. Logically, a feed mixture is fed to the first required catalyst, namely to a CO₂-to-methanol catalyst for use in step (ia). It is thus also logic that CO2 is actively fed in step (i). Step (i) can thus also be seen as a step of feeding a feed mixture comprising CO₂ (to a reaction zone) and converting a feed mixture comprising CO₂ into an intermediate mixture comprising CO and aromatics by (or in) steps (ia) and (ib).

In step (ia), carbon dioxide (CO₂) is at least partially, and preferably completely, converted into methanol (CH₃OH; or MeOH) and carbon monoxide (CO). The conversion occurs in the presence of a CO₂-to-methanol catalyst. As used herein, a CO₂-to-methanol catalyst promotes a methanol-forming reaction using CO₂ as a raw material. In other words, the CO₂-to-methanol catalyst lowers the activation energy of a respective methanol-forming reaction. As used herein, a methanol-forming reaction is a reaction which comprises a reaction of CO₂ with hydrogen (H₂) to yield CH₃OH. Such a methanol-forming reaction occurs in step (ia) which thus comprises the following reaction: CO₂ + 3H₂ → CH₃OH + H₂O

The above reaction may occur directly, or may be the sum of two partial reactions, i.e., of a first partial reaction: CO₂ + H₂ → CO + H₂O, and
a second partial reaction: CO + 2H₂ → CH₃OH

The CO₂-to-methanol catalyst can be used as such, or in supported form, or in admixture with other solids, for example in an admixture with a zeolite.

In step (ib), methanol from step (ia) is at least partially, and preferably completely, converted into aromatics. That is, a conversion of methanol to aromatics (MTA) occurs. As used herein, aromatics are aromatic in the sense of the IUPAC Gold Book. More specifically, aromatics are cyclically conjugated molecular entities with a stability (due to delocalization) significantly greater than that of a hypothetical localized structure (e.g., Kekulé structure). Such cyclically conjugated molecular entities have aromaticity. A method for determining aromaticity can in particular be the observation of diatropicity in the ¹H-NMR spectrum.

In step (ib), methanol from step (ia) is converted using a zeolite-based catalyst, that is, in the presence of a zeolite-based catalyst. As used herein, zeolite-based means that the catalyst comprises zeolite. The zeolite-based catalyst is preferably composed of ≥ 50 wt.%, more preferably of ≥ 60 wt.%, still more preferably of ≥ 70 wt.%, even more preferably of ≥ 80 wt.% and in particular preferably of ≥ 90 wt.% of zeolite; the weight percentages are based on the total weight of the zeolite-based catalyst. In a particular case, the zeolite-based catalyst consists of zeolite. As used herein, zeolite is given the same meaning as usual in the art. In particular, a zeolite has an aluminosilicate matrix with a tetrahedral arrangement of silicon (Si⁴⁺) and aluminium (Al³⁺) cations surrounded by four oxygen anions (O²⁻). This regularly results in a macromolecular three-dimensional structure of SiO₂ and AlO₂ tetrahedral building blocks. As the AlO₂ tetrahedral building blocks are negatively charged, zeolites regularly comprise additional charge-compensating cations, e.g., alkali metal cations, alkaline earth metal cations, protons and/or ammonia cations.

The particular synthetic pathways of the MTA reaction depend among others on the actually used zeolite and its structure. However, typical aromatics obtained in the MTA reaction are benzene, toluene and xylene as well as mixtures thereof. Step (ib) thus preferably yields at least one of benzene, toluene and xylene.

In step (ii), CO is at least partially, and preferably completely, converted into saturated hydrocarbons, and optionally into additional compounds, especially into additional unsaturated hydrocarbons. Saturated carbons are regularly linear, branched or cyclic alkyls. Unsaturated carbons are regularly linear, branched or cyclic alkenyls or alkynyls. Saturated and unsaturated hydrocarbons may comprise heteroatoms like oxygen (O), sulfur (S), nitrogen (N) or phosphorous (P). However, according to the present invention, the saturated and unsaturated hydrocarbons are preferably free from heteroatoms like oxygen (O), sulfur (S), nitrogen (N) and phosphorous (P).

In step (ii), the obtained saturated hydrocarbons comprise saturated C7+ hydrocarbons, preferably saturated C8+ hydrocarbons. Saturated C7+ hydrocarbons are saturated hydrocarbons which contain seven or more (≥7) carbon atoms. Saturated C8+ hydrocarbons are saturated hydrocarbons which contain eight or more (≥8) carbon atoms. Saturated C7+ hydrocarbons are particularly suitable for combustion in aircraft engines. This is because the C7+ hydrocarbons have a high volumetric energy density, a flash point above 30°C and a good autoignition temperature while maintaining good cold flow properties of the produced fuel. Appropriate cold flow properties can prevent fuel freezing at low temperatures of for example -40°C, which are typical for cruising altitudes of the powered aircrafts. Accordingly, when the saturated hydrocarbons comprise saturated C7+ hydrocarbons, the inventive method can be particularly suitable for producing sustainable fuel which meets the requirements for aviation fuel. The mentioned effect can be particularly pronounced when the saturated hydrocarbons comprise saturated C8+ hydrocarbons.

In step (ii), CO is converted using a Fischer-Tropsch catalyst (or FT-catalyst), that is, in the presence of a Fischer-Tropsch catalyst. As used herein, a Fischer-Tropsch catalyst promotes a Fischer-Tropsch reaction. In other words, the Fischer-Tropsch catalyst lowers the activation energy of a Fischer-Tropsch reaction. As used herein, a Fischer-Tropsch reaction is a reaction which comprises a reaction of CO with hydrogen (H₂) to yield saturated and optionally unsaturated hydrocarbons. During the reaction, the hydrocarbons regularly grow in a repeated sequence in which hydrogen atoms are added to carbon and oxygen, the C-O bond of CO is split and a new C-C bond is formed. For one -CH₂- group, the reaction can be given as follows:

CO + 2 H₂ → (CH₂) + H₂O

Such a Fischer-Tropsch reaction can for example comprise the following reaction steps:
associative adsorption of CO by the FT-catalyst;
splitting of the C-O bond;
dissociative adsorption of 2 H₂ by the FT-catalyst;
transfer of 2 H to the oxygen to yield H₂O
desorption of H₂O from the FT-catalyst;
transfer of 2 H to the carbon to yield CH₂.

In steps (ia), (ib) and (ii), the afore-listed catalysts are used, namely, a CO₂-to-methanol catalyst, a zeolite-based catalyst and a Fischer-Tropsch catalyst. According to the present invention, all these catalysts are used in solid form. Here, solid form refers to the aggregation state of the respective catalysts, in particular under normal conditions of 298.15 K and 101.3 kPa.

The method for producing sustainable fuel according to the present invention uses CO₂ as a feedstock, which is at least partially converted and is hence not emitted to the environment. The inventive method can thus help to reduce the CO₂ footprint associated with the produced sustainable fuel. Herein, the CO₂ footprint refers to the amount of carbon dioxide released into the atmosphere.

The method for producing sustainable fuel according to the present invention can be carried out in one or more reaction vessels, especially reactors, which have previously been used for conversion of fossil feedstock. The inventive method may thus allow for existing infrastructure to be maintained.

The method for producing sustainable fuel according to the present invention combines ways of producing aromatics and larger hydrocarbons and yields a sustainable fuel comprising such aromatics and larger hydrocarbons in one single continuous reaction sequence. The inventive method can thereby lead to improved energy efficiency and/or improved cost efficiency of the fuel production.

The method for producing sustainable fuel according to the present invention combines ways of producing aromatics and larger hydrocarbons and yields a sustainable fuel comprising such aromatics and larger hydrocarbons. The inventive method can thereby produce sustainable fuel which meets the requirements for aviation fuel.

It is preferred that in a method according to the present invention, in step (ii) CO is at least partially converted into unsaturated hydrocarbons. Such additional unsaturated hydrocarbons are prone to further oligomerisation and/or aromatisation. Accordingly, producing unsaturated hydrocarbons in step (ii) can further enhance the contents of larger hydrocarbons and/or aromatics in the finally obtained fuel, and can typically enhance the contents of both, larger hydrocarbons and aromatics. Hence, the additional production of unsaturated hydrocarbons in step (ii) can further contribute to the aviation fuel characteristics of the produced fuel. In this context, it is particularly preferred that the unsaturated hydrocarbons are recirculated to the zeolite-based catalyst of step (ib). In this way, the yield of aromatics obtained in step (ib) can be further increased. Such a preferred method leads to a sustainable fuel which has an increased aromatics content and which can hence regularly meet the requirements for aviation fuel.

It is preferred that in a method according to the present invention, the aromatics obtained in step (ib) are subsequently at least partially converted into C8+ aromatics, more preferably by alkylation with unsaturated C₂-C₆ hydrocarbons. As used herein, C8+ aromatics are aromatics which contain 8 or more carbons (or carbon atoms). C8+ aromatics are valuable components of aviation fuels. This is because the C8+ aromatics improve the cold flow properties of the produced fuel while resulting in appropriate swelling properties of the seals used in the aircrafts. Such improved cold flow properties can prevent fuel freezing at low temperatures of for example -40°C, which are typical for cruising altitudes of the powered aircrafts. Accordingly, when the hydrocarbons comprise C8+ aromatics, the inventive method can be particularly suitable for producing sustainable fuel which meets the requirements for aviation fuel. In other words, the subsequent conversion of the aromatics obtained in step (ib) into C8+ aromatics can make the fuel produced by the inventive method particularly rich in aromatics and hence also particularly suitable for aviation fuel.

It is preferred that in a method according to the present invention, in step (i) the CO₂ is at least partially reacted with H₂, and/or in step (ii) the CO is at least partially reacted with H₂. A reaction with H₂ in either step can lead to an increased conversion of CO₂ and/or CO, which can further reduce the CO₂ footprint. Additionally, an increased conversion of CO₂ and/or CO can further improve the energy efficiency and/or the cost efficiency of the fuel production.

It is preferred that in a method according to the present invention, the CO₂-to-methanol catalyst comprises a metal oxide compound, more preferably Cu/ZnO/Al₂O₃, Cr₂O₃, In₂O₃/ZrO₂, or ZnOZrO₂. The use of a CO₂-to-methanol catalyst which comprises a metal oxide compound can lead to an increased conversion of CO₂ in step (i), which can further reduce the CO₂ footprint. Additionally, such a CO₂-to-methanol catalyst comprising a metal oxide compound may already be used in existing infrastructure. Hence, such a CO₂-to-methanol catalyst comprising a metal oxide compound can help to maintain existing infrastructure. The mentioned effects can be particularly pronounced when the CO₂-to-methanol catalyst comprises Cu/ZnO/Al₂O₃, Cr₂O₃, In₂O₃/ZrO₂, or ZnOZrO₂.

It is preferred that in a method according to the present invention, the zeolite-based catalyst in step (ib) comprises an MFI-type zeolite (especially a ZSM-5 zeolite or an HZSM-5 zeolite), a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof. More preferably, the zeolite-based catalyst in step (ib) comprises a ZSM-5 zeolite or an HZSM-5 zeolite, particularly preferable an HZSM-5 zeolite (an HZSM-5 zeolite is a proton-exchanged form of a ZSM-5-type zeolite). The listed zeolite types are indicated here by the codes attributed by the International Zeolite Association. The use of a zeolite-based catalyst which comprises a zeolite of the listed types can lead to an increased conversion of methanol into aromatics, which can further improve the energy efficiency and/or the cost efficiency of the fuel production. Additionally, such a zeolite-based catalyst of the listed types may already be used in existing infrastructure. Hence, such a zeolite-based catalyst of the listed types can help to maintain existing infrastructure. The mentioned effects can be particularly pronounced when the zeolite-based catalyst in step (ib) comprises a ZSM-5 zeolite or an HZSM-5 zeolite, in particular an HZSM zeolite. Herein, a ZSM-5 zeolite and an HZSM-5 zeolite may be combinedly referred to as (H)ZSM-5 zeolite.

It is preferred that in a method according to the present invention, the zeolite-based catalyst in step (ib) comprises a metal-modified zeolite. As used herein, "metal-modified" means that the zeolite contains metal cations different from alkali metal cations and alkaline earth metal cations. Preferred metal cations are Zn-cations, Ga-cations, Ag-cations, Mo-cations and/or Re-cations. Accordingly, it is particularly preferred that the zeolite-based catalyst in step (ib) comprises a Zn-modified zeolite, a Ga-modified zeolite, an Ag-modified zeolite, an Mo-modified zeolite and/or a Re-modified zeolite. The use of such a metal-modified zeolite can lead to an even further increased conversion of methanol into aromatics, which can further improve the energy efficiency and/or the cost efficiency of the fuel production. Additionally, such a metal-modified zeolite may already be used in existing infrastructure. Hence, such a metal-modified zeolite can help to maintain existing infrastructure.

It is preferred that in a method according to the present invention, the Fischer-Tropsch catalyst comprises Fe and/or Co, more preferably Co. It is more preferred that the Fischer-Tropsch catalyst comprises Fe or Co which is supported on an oxide, in particular Co supported on an oxide. The use of a Fischer-Tropsch catalyst which comprises Fe or Co can lead to an increased conversion of CO in step (ii), which can further improve the energy efficiency and/or the cost efficiency of the fuel production. Additionally, such a Fischer-Tropsch catalyst comprising Fe or Co may already be used in existing infrastructure. Hence, such a Fischer-Tropsch catalyst comprising Fe or Co can help to maintain existing infrastructure. The mentioned effects can be particularly pronounced when the Fischer-Tropsch catalyst comprises Co. Optionally, the Fe comprised by the Fischer-Tropsch catalyst is metallic Fe, the Co comprised by the Fischer-Tropsch catalyst is metallic Co, or the Fe comprised by the Fischer-Tropsch catalyst is metallic Fe and the Co comprised by the Fischer-Tropsch catalyst is metallic Co.

It is preferred that in a method according to the present invention, CO from step (i) is additionally converted in step (ii) into C₁-C₆ hydrocarbons using the Fischer-Tropsch catalyst, followed by a step (iii) of converting C₁-C₆ hydrocarbons from step (ii) into aromatics using another zeolite-based catalyst. The "another" zeolite-based catalyst is different from the zeolite-based catalyst used in step (ib), i.e., it is a further zeolite-based catalyst. When additional C₁-C₆ hydrocarbons are produced from CO in step (ii), which C₁-C₆ hydrocarbons are subsequently converted in a step (iii) into aromatics over the further zeolite-based catalyst, the overall yield of aromatics of the method can be improved. Such an improved yield of aromatics can further help to meet the requirements for aviation fuel.

C₁-C₆ hydrocarbons are compounds containing at least one and at most six carbon atoms, i.e., 1, 2, 3, 4, 5 or 6 carbon atoms, and additionally hydrogen. The C₁-C₆ hydrocarbons can be saturated and/or unsaturated hydrocarbons. Saturated C₁-C₆ hydrocarbons are regularly linear, branched or cyclic alkyls. Unsaturated C₁-C₆ hydrocarbons are regularly linear, branched or cyclic alkenyls or alkynyls. The C₁-C₆ hydrocarbons may comprise heteroatoms like oxygen (O), sulfur (S), nitrogen (N) or phosphorous (P). However, according to the present invention, the C₁-C₆ hydrocarbons are preferably free from heteroatoms like oxygen (O), sulfur (S), nitrogen (N) and phosphorous (P).

It is preferred that in a method according to the present invention, the C₁-C₆ hydrocarbons produced in step (ii) comprise unsaturated hydrocarbons, more preferably alkenyls, still more preferably C₂-C₄ alkenyls. When the C₁-C₆ hydrocarbons comprise such unsaturated hydrocarbons, the subsequent conversion thereof into aromatics in step (iii) can be particularly effective.

It is more preferred that in a method according to the present invention, the C₁-C₆ hydrocarbons comprise ethylene, propylene and/or butylene. When the C₁-C₆ hydrocarbons produced in step (ii) comprise ethylene, propylene and/or butylene, the subsequent conversion thereof into aromatics in step (iii) can have a higher conversion rate and/or may require less energy. Accordingly, when the C₁-C₆ hydrocarbons comprise ethylene, propylene and/or butylene, the energy efficiency and/or the cost efficiency of the fuel production can be improved.

It is particularly preferred that the zeolite-based catalyst used in step (iii) comprises a metal-modified zeolite, preferably a Zn-modified zeolite, a Ga-modified zeolite, an Ag-modified zeolite, an Mo-modified zeolite and/or a Re-modified zeolite. The metal-modified zeolite can be an MFI-type zeolite, a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof. The use of a metal-modified zeolite can lead to an increased conversion of C₁-C₆ hydrocarbons into aromatics in step (iii), which can further improve the energy efficiency and/or the cost efficiency of the fuel production. Additionally, such a Zn-modified zeolite may already be used in existing infrastructure. Hence, such a Zn-modified zeolite can help to maintain existing infrastructure.

It is preferred that in a method according to the present invention, methane (CH₄) is produced in step (i) and/or in step (ii), which is subsequently at least partially converted into aromatics in step (iii). This additional synthesis of aromatics in the inventive method can improve the aromatics content of the proceeded sustainable fuel, which can further help to meet the requirements for aviation fuel. Moreover, the CH₄ from step (i) and/or step (ii) is not lost, but is rather valorised.

It is preferred that in a method according to the present invention, step (i), i.e., step (ia) and/or step (ib), is performed at a temperature of 200 to 550°C, more preferably of 200 to 450°C and still more preferably of 250 to 350°C. An increased temperature of 200 to 550°C in step (i) can lead to an increased conversion of the CO₂. This can help to further reduce the CO₂ footprint of the sustainable fuel produced by the inventive method. At the same time, too high temperatures may lead to a reduced energy efficiency and/or cost efficiency of the fuel production. Temperatures of 200 to 450°C and in particular of 250 to 350°C are therefore particularly preferred.

It is preferred that in a method according to the present invention, step (i), i.e., step (ia) and/or step (ib), is performed at an absolute pressure of 0.1 to 10 MPa, more preferably of 1 to 4 MPa. An absolute pressure of 0.1 to 10 MPa in step (i) can lead to an increased conversion of the CO₂. This can help to further reduce the CO₂ footprint of the sustainable fuel produced by the inventive method. At the same time, too high pressures may lead to a reduced energy efficiency and/or cost efficiency of the fuel production. Absolute pressures of 1 to 4 MPa are therefore particularly preferred.

It is preferred that in a method according to the present invention, the feed mixture in step (i) has a molar ratio of hydrogen to carbon oxides, i.e., a molar ratio H₂:COₓ, of 0.5 to 20, more preferably of 1 to 8, with x being 1 and/or 2, with x preferably being 2. With such a molar ratio of hydrogen to carbon oxides of 0.5 to 20 an increased conversion of CO₂ in step (i) may be achieved which can help to further reduce the CO₂ footprint of the sustainable fuel produced by the inventive method. These effects can be particularly pronounced when the ratio of hydrogen to carbon oxides is 1 to 8.

It is preferred that a method according to the present invention further comprises a cooling step in which CO from step (i) is cooled before being converted in step (ii). Such a cooling step can lower heating requirements for the reaction vessel, like a reactor, in which step (ii) is carried out, without however disadvantageously reducing the conversion rate of CO in step (ii). Such a cooling step can thus further improve the energy efficiency and/or the cost efficiency of the fuel production.

In the context of the cooling step, it is especially preferred that step (i) is carried out in a first reactor and that step (ii) is carried out in a physically separated second reactor, wherein the cooling takes place between the CO leaving the first reactor and the CO entering the second reactor.

It is preferred that in a method according to the present invention, H₂O is produced in step (i) to yield an intermediate mixture which additionally comprises H₂O, wherein the H₂O is at least partially separated from the intermediate mixture in the cooling step. The separation of H₂O can help to maintain or even increase the conversion rate of CO in step (ii). The separation of H₂O can thus further improve the energy efficiency and/or the cost efficiency of the fuel production.

It is preferred that in a method according to the present invention, the feed mixture is substantially free of CO. For example, the feed mixture may preferably contain less than 0.1 mol% CO, more preferably less than 0.01 mol% CO, respectively based on the total composition of the feed mixture. When the feed mixture is substantially free of CO, preferably free of CO, the absent CO cannot interfere with the MTA reaction. Accordingly, a substantial absence of CO and in particular a complete absence of CO in the feed mixture can lead to an increased conversion of CO₂ in step (i), which can further reduce the CO₂ footprint.

It is preferred that in a method according to the present invention, step (ii) is performed at a temperature of 150 to 500°C, more preferably of 200 to 300°C. An increased temperature of 150 to 500°C in step (ii) can lead to an increased conversion of the CO. At the same time, too high temperatures may lead to a reduced energy efficiency and/or cost efficiency of the fuel production. A temperature of 200 to 300°C is therefore particularly preferred.

It is preferred that in a method according to the present invention, step (ii) is performed at an absolute pressure of 0.1 to 5 MPa, more preferably of 1 to 3 MPa. An absolute pressure of 0.1 to 5 MPa in step (ii) can lead to an increased conversion of the CO. At the same time, too high pressures may lead to a reduced energy efficiency and/or cost efficiency of the fuel production. Absolute pressures of 1 to 3 MPa are therefore particularly preferred.

It is preferred that in a method according to the present invention,
in step (i) CO₂ comprised by the feed mixture is reacted with H₂ at a temperature of 200 to 550°C and under an absolute pressure of 0.1 to 10 MPa to yield an intermediate mixture comprising CO, aromatics and H₂O by
(ia) converting the CO₂ at least partially into methanol, CO and H₂O using a CO₂-to-methanol catalyst which comprises a metal oxide compound, and
(ib) converting methanol from step (ia) at least partially into aromatics using a zeolite-based catalyst which comprises an MFI-type zeolite,
wherein the H₂O and/or the aromatics is/are at least partially separated from the intermediate mixture in a subsequent cooling step before CO is converted in step (ii),
wherein in step (ii) CO from step (i) is reacted with H₂ using a Fischer-Tropsch catalyst which comprises Co to yield saturated C8+ hydrocarbons and C₁-C₆ hydrocarbons, and
wherein the method further comprises a step (iii) in which C₁-C₆ hydrocarbons from step (ii) are converted into aromatics using another zeolite-based catalyst which comprises a metal-modified zeolite.

Such a preferred method for producing sustainable fuel according to the present invention can in particular help to reduce the CO₂ footprint associated with the produced sustainable fuel, may allow for existing infrastructure to be maintained, can lead to an improved energy efficiency and/or an improved cost efficiency of the fuel production and can produce sustainable fuel which meets the requirements for aviation fuel.

Disclosed is also a use of sustainable fuel obtained by a method as described herein, which fuel comprises aromatics obtained in step (ib) and saturated C7+ hydrocarbons obtained in step (ii), as aviation fuel. Any use of sustainable fuel obtained by a method as described herein as aviation fuel may also be considered as a corresponding method of using such sustainable fuel as aviation fuel following a method as described herein. In other words, a use can also be seen as a method according to the present invention which comprises another step after step (ii), which another step is a step of using aromatics obtained in step (ib) and saturated C7+ hydrocarbons obtained in step (ii) together as aviation fuel.

Also disclosed herein is a sustainable fuel obtainable by a method as described herein. The preferred embodiments of the method described herein including the claims are likewise preferred for this sustainable fuel in an analogous manner.

### Brief description of the drawings

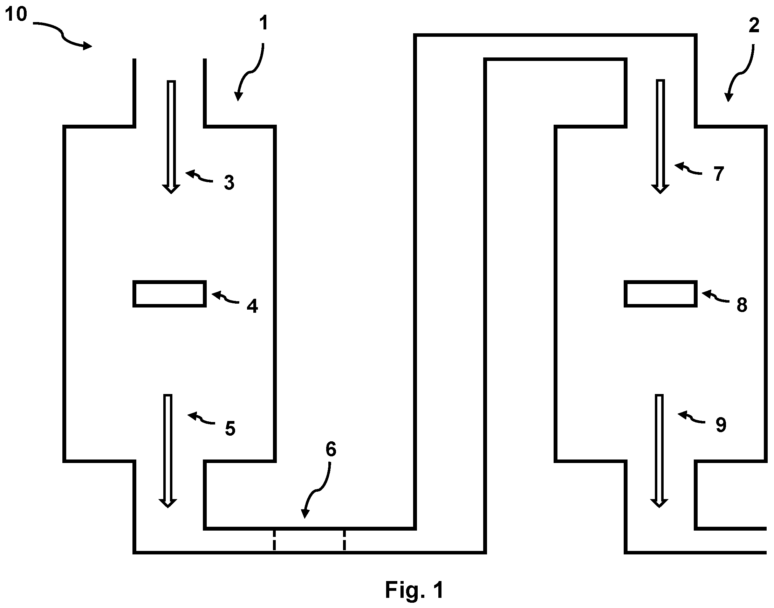

Fig. 1 shows an exemplary reactor system in which a method according to the present invention can be carried out.

### Exemplary embodiment 1

The present invention is further described with reference to the accompanying Fig. 1 which shows an exemplary reactor system 10. The reactor system 10 has two reactors, namely a first reactor 1 and a second reactor 2, which are in fluid communication with each other. A first feed 3 is fed to the first reactor 1. Feed 3 comprises CO₂ and H₂, and depending on the source of the CO₂ potentially minor amounts of CO (typically however 0% CO) and light hydrocarbons. The first reactor 1 contains a first catalyst bed 4 which contains a metal oxide compound as a CO₂-to-methanol catalyst and an MFI-type zeolite as a zeolite-based catalyst. The first reactor 1 is operated at elevated temperatures between 200 and 550°C and absolute pressures between 0.1 and 10 MPa. In the first reactor 1, methanol and CO are produced using the metal oxide compound, and aromatics are produced from the synthesized methanol using the MFI-type zeolite. In addition to the aromatics, light olefins (C₂-C₄ olefins or alkenyls) may also be produced due to the presence of the MFI-type zeolite. A further side product generated in the first reactor 1 is water (H₂O). Methane (CH₄) in low amounts may also be produced in reactor 1. Overall, a first product 5 is produced in the first reactor 1 which contains CO and aromatics, further regularly contains H₂O, unreacted CO₂, unreacted H₂, unreacted methanol and typically light olefins and low amounts of CH₄. The first product 5 is withdrawn from reactor 1 and is sent to reactor 2. In-between reactor 1 and reactor 2, a cooling device 6 may be arranged. The cooling device 6 cools the first product 5 to the reaction temperature of the second reactor 2. The cooling device 6 further separates at least some of the H₂O produced in the first reactor 1 to give the second feed 7 which is fed to the second reactor 2. Apart from separating H₂O, cooling device 6 can also separate out methanol and aromatics. The second feed 7 then contains CO, CO₂, H₂, light olefins and CH₄. The second reactor 2 contains a second catalyst bed 8 which contains a Co-based Fischer-Tropsch catalyst and an MFI-type zeolite catalyst. The second reactor 2 is operated at elevated temperatures between 200 and 300°C and absolute pressures between 0.1 and 5 MPa. In the second reactor 2, unreacted H₂ and CO generated in the first reactor are converted by the Fischer-Tropsch catalyst to long hydrocarbons, including saturated C7+ hydrocarbons and usually saturated C8+ hydrocarbons, and unsaturated hydrocarbons, usually light olefins (C₂-C₄ olefins). The light olefins are converted to aromatics by the MFI-type zeolite catalyst. As a result, a second product 9 is obtained which contains long hydrocarbons and aromatics, and potentially residual CO, H₂, CO₂ and/or CH₄. The second product 9 can be withdrawn from the second reactor as an intermediate product, or as the final product. In case aromatics produced in the first reactor 1 are separated from the first product 5, they can be admixed with the second product 9 to give a final product of higher aromatics content. The final product can in either case be used as a sustainable (raw) fuel for further use or refinement thereof.

### Exemplary embodiment 2

In this additional embodiment, the unsaturated hydrocarbons, especially C₂-C₄ olefins, synthesized in the second reactor over the Fischer-Tropsch catalyst, are not converted to aromatics in the second reactor, but are separated. Subsequently, the unsaturated hydrocarbons are recirculated to the first catalyst bed 4 in the first reactor 1, more specifically to the MFI-type zeolite in reactor 1. An additional conversion of the recirculated unsaturated hydrocarbons into aromatics over the MFI-type zeolite in reactor 1 takes place. In this way, the overall aromatics content is further increased compared to Embodiment 1.

### Exemplary embodiment 3

In this further embodiment, the aromatics obtained in step (ib) contained in the second product 9 predominantly comprise benzene, toluene and xylene, i.e., C6+ aromatics. These C6+ aromatics are produced according to the reaction sequence of Embodiment 1. Subsequently, unsaturated C₂-C₆ hydrocarbons are fed to and reacted with the C6+ aromatics. Thereby, the C6+ aromatics are converted into C8+ aromatics by alkylation with the unsaturated C₂-C₆ hydrocarbons. In this way, the content of higher aromatics, namely C8+ aromatics, is further increased compared to Embodiment 1.

### Exemplary embodiment 4

In this further embodiment, the aromatics obtained in step (ib) contained in the second product 9 predominantly comprise benzene, toluene and xylene, i.e., C6+ aromatics. These C6+ aromatics are produced according to the reaction sequence of Embodiment 2. Subsequently, unsaturated C₂-C₆ hydrocarbons are fed to and reacted with the C6+ aromatics. Thereby, the C6+ aromatics are converted into C8+ aromatics by alkylation with the unsaturated C₂-C₆ hydrocarbons. In this way, the content of higher aromatics, namely C8+ aromatics, is further increased compared to Embodiment 2.

### List of reference signs

- 1:: First reactor
- 2:: Second reactor
- 3:: First feed
- 4:: First catalyst bed
- 5:: First product
- 6:: Cooling device
- 7:: Second feed
- 8:: Second catalyst bed
- 9:: Second product
- 10:: Reactor system

## Claims

1. A method for producing sustainable fuel, comprising the steps:
(i) converting a feed mixture comprising CO₂ into an intermediate mixture comprising CO and aromatics by
(ia) converting the CO₂ at least partially into methanol and CO using a CO₂-to-methanol catalyst, and
(ib) converting methanol from step (ia) at least partially into aromatics using a zeolite-based catalyst, and
(ii) converting CO from step (ia) at least partially into saturated hydrocarbons using a Fischer-Tropsch catalyst, wherein the saturated hydrocarbons comprise saturated C7+ hydrocarbons.

2. The method according to claim 1, wherein in step (ii) CO is at least partially converted into unsaturated hydrocarbons.

3. The method according to claim 2, wherein the unsaturated hydrocarbons are recirculated to the zeolite-based catalyst of step (ib).

4. The method according to any preceding claim, wherein the aromatics obtained in step (ib) are subsequently at least partially converted into C8+ aromatics, preferably by alkylation with unsaturated C₂-C₆ hydrocarbons.

5. The method according to any of the preceding claims, wherein the CO₂-to-methanol catalyst comprises a metal oxide compound, preferably Cu/ZnO/Al₂O₃, Cr₂O₃, In₂O₃/ZrO₂, or ZnOZrO₂.

6. The method according to any of the preceding claims, wherein the zeolite-based catalyst in step (ib) comprises an MFI-type zeolite, a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof.

7. The method according to any of the preceding claims, wherein the Fischer-Tropsch catalyst comprises Fe and/or Co, preferably Co.

8. The method according to any of the preceding claims, wherein in step (ii) CO from step (i) is additionally converted into C₁-C₆ hydrocarbons using the Fischer-Tropsch catalyst, the method further comprising a step
(iii) converting C₁-C₆ hydrocarbons from step (ii) into aromatics using another zeolite-based catalyst, wherein the zeolite-based catalyst used in step (iii) preferably comprises a metal-modified zeolite.

9. The method according to any of the preceding claims, wherein the saturated hydrocarbons comprise saturated C8+ hydrocarbons.

10. The method according to any of the preceding claims, wherein step (i) is performed at a temperature of 200 to 550°C and/or under an absolute pressure of 0.1 to 10 MPa.

11. The method according to any of the preceding claims, further comprising a cooling step in which CO from step (i) is cooled before being converted in step (ii).

12. The method according to claim 11, wherein H₂O is produced in step (i) to yield an intermediate mixture additionally comprising H₂O, wherein the H₂O is at least partially separated from the intermediate mixture in the cooling step.

13. The method according to any of the preceding claims, wherein the feed mixture is substantially free of CO.

14. The method according to any of the preceding claims,
wherein in step (i) CO₂ comprised by the feed mixture is reacted with H₂ at a temperature of 200 to 550°C and under an absolute pressure of 0.1 to 10 MPa to yield an intermediate mixture comprising CO, aromatics and H₂O by
(ia) converting the CO₂ at least partially into methanol, CO and H₂O using a CO₂-to-methanol catalyst which comprises a metal oxide compound, and
(ib) converting methanol from step (ia) at least partially into aromatics using a zeolite-based catalyst which comprises an MFI-type zeolite,
wherein the H₂O and/or the aromatics is/are at least partially separated from the intermediate mixture in a subsequent cooling step before CO is converted in step (ii),
wherein in step (ii) CO from step (i) is reacted with H₂ using a Fischer-Tropsch catalyst which comprises Co to yield saturated C8+ hydrocarbons and C₁-C₆ hydrocarbons,
the method further comprising a step (iii) in which C₁-C₆ hydrocarbons from step (ii) are converted into aromatics using another zeolite-based catalyst which comprises a metal-modified zeolite.

15. The method according to any of claims 1 to 14, which comprises another step after step (ii), which another step is a step of using aromatics obtained in step (ib) and saturated C7+ hydrocarbons obtained in step (ii) together as aviation fuel.

## Patentansprüche

1. Verfahren zum Produzieren von nachhaltigem Kraftstoff, die folgenden Schritte umfassend:
(i) Umwandeln einer Beschickungsmischung, die CO₂ umfasst, in eine Zwischenmischung, die CO und Aromate umfasst, durch
(ia) Umwandeln des CO₂ mindestens teilweise in Methanol und CO unter Verwendung eines CO₂-zu-Methanol-Katalysators, und
(ib) Umwandeln des Methanols aus Schritt (ia) mindestens teilweise in Aromate unter Verwendung eines Zeolith-basierten-Katalysators, und
(ii) Umwandeln des CO aus Schritt (ia) mindestens teilweise in gesättigte Kohlenwasserstoffe unter Verwendung eines Fischer-Tropsch-Katalysators, wobei die gesättigten Kohlenwasserstoffe gesättigte C7+-Kohlenwasserstoffe umfassen.

2. Verfahren nach Anspruch 1, wobei in Schritt (ii) CO mindestens teilweise in ungesättigte Kohlenwasserstoffe umgewandelt wird.

3. Verfahren nach Anspruch 2, wobei die ungesättigten Kohlenwasserstoffe dem Zeolith-basierten Katalysator aus Schritt (ib) wieder zugeführt werden.

4. Verfahren nach einem der vorhergehenden Ansprüche, wobei die in Schritt (ib) erhaltenen Aromaten anschließend mindestens teilweise in C8+-Aromate, bevorzugt durch Alkylierung mit ungesättigten C₂-C₆-Kohlenwasserstoffen, umgewandelt werden.

5. Verfahren nach einem der vorhergehenden Ansprüche, wobei der CO₂-zu-Methanol-Katalysator eine Metalloxidverbindung umfasst, bevorzugt Cu/ZnO/Al₂O₃, Cr₂O₃, In₂O₃/ZrO₂, oder ZnOZrO₂.

6. Verfahren nach einem der vorhergehenden Ansprüche, wobei der Zeolith-basierte Katalysator in Schritt (ib) einen MFI-Typ-Zeolith, einen CHA-Typ-Zeolith, einen BEA-Typ-Zeolith, einen MOR-Typ-Zeolith, einen FAU-Typ-Zeolith, einen MEL-Typ-Zeolith, einen FER-Typ-Zeolith, einen MTT-Typ-Zeolith, einen TON-Typ-Zeolith, einen ERI-Typ-Zeolith, einen MTW-Typ-Zeolith, einen MWW-Typ-Zeolith oder Mischungen derselben umfasst.

7. Verfahren nach einem der vorhergehenden Ansprüche, wobei der Fischer-Tropsch-Katalysator Fe und/oder Co, bevorzugt Co, umfasst.

8. Verfahren nach einem der vorhergehenden Ansprüche, wobei in Schritt (ii) CO aus Schritt (i) unter Verwendung des Fischer-Tropsch-Katalysators zusätzlich in C₁-C₆ Kohlenwasserstoffe umgewandelt wird, das Verfahren ferner den folgenden Schritt umfassend:
(iii) Umwandeln von C₁-C₆-Kohlenwasserstoffen aus Schritt (ii) in Aromate unter Verwendung eines anderen Zeolith-basierten Katalysators, wobei der in Schritt (iii) verwendete Zeolith-basierte Katalysator bevorzugt einen Metall-modifizierten Zeolith umfasst.

9. Verfahren nach einem der vorhergehenden Ansprüche, wobei die gesättigten Kohlenwasserstoffe gesättigte C8+-Kohlenwasserstoffe umfassen.

10. Verfahren nach einem der vorhergehenden Ansprüche, wobei Schritt (i) bei einer Temperatur von 200 bis 550 °C und/oder unter einem absoluten Druck von 0,1 bis 10 MPa durchgeführt wird.

11. Verfahren nach einem der vorhergehenden Ansprüche, ferner umfassend einen Kühlschritt, in dem CO aus Schritt (i) abgekühlt wird, bevor es in Schritt (ii) umgewandelt wird.

12. Verfahren nach Anspruch 11, wobei H₂O in Schritt (i) produziert wird, um eine Zwischenmischung hervorzubringen, die zusätzlich H₂O umfasst, wobei das H₂O in dem Kühlschritt mindestens teilweise von der Zwischenmischung getrennt wird.

13. Verfahren nach einem der vorhergehenden Ansprüche, wobei die Beschickungsmischung im Wesentlichen frei von CO ist.

14. Verfahren nach einem der vorhergehenden Ansprüche,
wobei in Schritt (i) in der Beschickungsmischung umfasstes CO₂ bei einer Temperatur von 200 bis 550 °C und unter einem absoluten Druck von 0,1 bis 10 MPa mit H₂ reagiert wird, um eine Zwischenmischung hervorzubringen, die CO, Aromate und H₂O umfasst, durch
(ia) Umwandeln des CO₂ mindestens teilweise in Methanol, CO und H₂O unter Verwendung eines CO₂-zu-Methanol-Katalysators, der eine Metall-Oxid-Verbindung umfasst, und
(ib) Umwandeln des Methanols aus Schritt (ia) mindestens teilweise in Aromate unter Verwendung eines Zeolith-basierten-Katalysators, der einen MFI-Typ-Zeolith umfasst,
wobei das H₂O und/oder die Aromaten mindestens teilweise von der Zwischenmischung in einem anschließenden Kühlschritt getrennt wird/werden, bevor CO in Schritt (ii) umgewandelt wird,
wobei in Schritt (ii) CO aus Schritt (i) mit H₂ reagiert wird, unter Verwendung eines Fischer-Tropsch-Katalysators, der Co umfasst, um gesättigte C8+-Kohlenwasserstoffe und C₁-C₆-Kohlenwasserstoffe hervorzubringen,
wobei das Verfahren ferner einen Schritt (iii) umfasst, in dem C₁-C₆-Kohlenwasserstoffe aus Schritt (ii) unter Verwendung eines anderen Zeolith-basierten Katalysators, der einen Metall-modifizierten Zeolith umfasst, in Aromate umgewandelt werden.

15. Verfahren nach einem der Ansprüche 1 bis 14, das einen anderen Schritt nach Schritt (ii) umfasst, wobei der andere Schritte ein Schritt des Verwendens von in Schritt (ib) erhaltenen Aromaten und in Schritt (ii) erhaltenen C7+-Kohlenwasserstoffen zusammen als Flugzeugkraftstoff ist.

## Revendications

1. Procédé de production de carburant durable, comprenant les étapes de :
(i) conversion d'un mélange d'alimentation comprenant du CO₂ en un mélange intermédiaire comprenant du CO et des composés aromatiques, par
(ia) conversion du CO₂ au moins partiellement en méthanol et en CO au moyen d'un catalyseur de conversion de CO₂ en méthanol, et
(ib) conversion du méthanol provenant de l'étape (ia) au moins partiellement en composés aromatiques au moyen d'un catalyseur à base de zéolithe, et
(ii) conversion du CO provenant de l'étape (ia) au moins partiellement en hydrocarbures saturés au moyen d'un catalyseur de Fischer-Tropsch, dans lequel les hydrocarbures saturés comprennent des hydrocarbures saturés C7+.

2. Procédé selon la revendication 1, dans lequel, à l'étape (ii), le CO est au moins partiellement converti en hydrocarbures insaturés.

3. Procédé selon la revendication 2, dans lequel les hydrocarbures insaturés sont recyclés vers le catalyseur à base de zéolithe de l'étape (ib).

4. Procédé selon l'une quelconque des revendications précédentes, dans lequel les composés aromatiques obtenus à l'étape (ib) sont ensuite au moins partiellement convertis en composés aromatiques C8+, de préférence par alkylation avec des hydrocarbures insaturés C₂-C₆.

5. Procédé selon l'une quelconque des revendications précédentes, dans lequel le catalyseur de conversion de CO₂ en méthanol comprend un composé d'oxyde métallique, de préférence Cu/ZnO/Al₂O₃, Cr₂O₃, In₂O₃/ZrO₂, ou ZnOZrO₂.

6. Procédé selon l'une quelconque des revendications précédentes, dans lequel le catalyseur à base de zéolithe de l'étape (ib) comprend une zéolithe de type MFI, une zéolithe de type CHA, une zéolithe de type BEA, une zéolithe de type MOR, une zéolithe de type FAU, une zéolithe de type MEL, une zéolithe de type FER, une zéolithe de type MTT, une zéolithe de type TON, une zéolithe de type ERI, une zéolithe de type MTW, une zéolithe de type MWW ou un mélange de celles-ci.

7. Procédé selon l'une quelconque des revendications précédentes, dans lequel le catalyseur de Fischer-Tropsch comprend du Fe et/ou du Co, de préférence du Co.

8. Procédé selon l'une quelconque des revendications précédentes, dans lequel, à l'étape (ii), le CO provenant de l'étape (i) est également converti en hydrocarbures C₁-C₆ au moyen du catalyseur de Fischer-Tropsch, le procédé comprenant en outre une étape de
(iii) conversion d'hydrocarbures C₁-C₆ provenant de l'étape (ii) en composés aromatiques au moyen d'un autre catalyseur à base de zéolithe, dans lequel le catalyseur à base de zéolithe utilisé à l'étape (iii) comprend de préférence une zéolithe modifiée par un métal.

9. Procédé selon l'une quelconque des revendications précédentes, dans lequel les hydrocarbures saturés comprennent des hydrocarbures saturés C8+.

10. Procédé selon l'une quelconque des revendications précédentes, dans lequel l'étape (i) est effectuée à une température comprise entre 200 et 550 °C et/ou sous une pression absolue de 0,1 à 10 MPa.

11. Procédé selon l'une quelconque des revendications précédentes, comprenant en outre une étape de refroidissement, dans laquelle le CO provenant de l'étape (i) est refroidi avant d'être converti à l'étape (ii).

12. Procédé selon la revendication 11, dans lequel de l'H₂O est produite à l'étape (i), de manière à obtenir un mélange intermédiaire comprenant également de l'H₂O, dans lequel l'H₂O est au moins partiellement séparée du mélange intermédiaire lors de l'étape de refroidissement.

13. Procédé selon l'une quelconque des revendications précédentes, dans lequel le mélange d'alimentation est sensiblement exempt de CO.

14. Procédé selon l'une quelconque des revendications précédentes,
dans lequel, à l'étape (i), du CO₂ contenu dans le mélange d'alimentation est mis en réaction avec du H₂ à une température comprise entre 200 et 550 °C et sous une pression absolue de 0,1 à 10 MPa, de manière à obtenir un mélange intermédiaire comprenant du CO, des composés aromatiques et de l'H₂O par
(ia) conversion du CO₂ au moins partiellement en méthanol, CO et H₂O au moyen d'un catalyseur de conversion de CO₂ en méthanol comprenant un composé d'oxyde métallique, et
(ib) conversion du méthanol provenant de l'étape (ia) au moins partiellement en composés aromatiques au moyen d'un catalyseur à base de zéolithe comprenant une zéolithe de type MFI,
dans lequel l'H₂O et/ou les composés aromatiques sont au moins partiellement séparés du mélange intermédiaire lors d'une étape de refroidissement ultérieure avant que le CO ne soit converti à l'étape (ii),
dans lequel, à l'étape (ii), le CO provenant de l'étape (i) est mis en réaction avec du H₂ au moyen d'un catalyseur de Fischer-Tropsch comprenant du Co, de manière à obtenir des hydrocarbures saturés C8+ et des hydrocarbures C₁-C₆,
le procédé comprenant en outre une étape (iii) dans laquelle les hydrocarbures C₁-C₆ provenant de l'étape (ii) sont convertis en composés aromatiques au moyen d'un autre catalyseur à base de zéolithe comprenant une zéolithe modifiée par un métal.

15. Procédé selon l'une quelconque des revendications 1 à 14, comprenant une autre étape après l'étape (ii), laquelle autre étape est une étape consistant à utiliser conjointement les composés aromatiques obtenus à l'étape (ib) et les hydrocarbures saturés C7+ obtenus à l'étape (ii) comme carburant d'aviation.
